# EUROPÄISCHE PATENTSCHRIFT

(11) **EP 0 832 195 B1**
(45) Veröffentlichungstag und Bekanntmachung des Hinweises auf die Patenterteilung: **09.11.2005**
(21) Anmeldenummer: 96917341.8
(22) Anmeldetag: 07.06.1996
(51) Int. Cl.: C12N 5/08, G01N 33/50, C12Q 1/02

(54) **VERFAHREN ZUR OSTEOPOROSEDIAGNOSE UND ZUR TESTUNG POTENTIELLER OSTEOPOROSE-THERAPEUTIKA UNTER VERWENDUNG VON STANDARDISIERTEN, PRIMÄREN OSTEOBLASTENZELLKULTUREN AUS OSTEOPOROTISCHEN PATIENTEN**
METHOD OF DIAGNOSIS OF OSTEOPOROSIS AND FOR TESTING POTENTIAL OSTEOPOROSIS THERAPEUTIC AGENTS USING STANDARDIZED, PRIMARY OSTEOBLAST CELL CULTURES TAKEN FROM PATIENTS SUFFERING FROM OSTEOPOROSIS
METHODE POUR LES DIAGNOSTICS D'OSTEOPOROSE ET LES TESTS DE TRAITEMENTS DE L'OSTEOPOROSE EN UTILISANT CULTURES DE CELLULES OSTEOBLASTES PRIMAIRES ET STANDARDISEES PRELEVEES SUR DES PATIENTS ATTEINTS D'OSTEOPOROSE

(30) Priorität: 07.06.1995 DE 19521942; 05.01.1996 DE 19601052
(43) Veröffentlichungstag der Anmeldung: 01.04.1998
(73) Patentinhaber: Co.Don Aktiengesellschaft, 14513 Teltow (DE)
(72) Erfinder: JOSIMOVIC-ALASEVIC, Olivera, D-10717 Berlin (DE); FRITSCH, Karl-Gerd, D-14957 Berlin (DE); ITTNER, Jochen, D-86159 Augsburg (DE)
(74) Vertreter: Ziebig, Marlene
(86) Internationale Anmeldenummer: PCT/DE1996/001042
(87) Internationale Veröffentlichungsnummer: WO 1996/040873

(56) Entgegenhaltungen:
- THE JOURNAL OF CLINICAL INVESTIGATION, Bd. 88, 1991, Seiten 1167-1172, XP000605181 PIERRE J. MARIE: "Decreased DNA synthesis by cultured osteoblastic cells in eugonadal osteoporotic men with defective bone formation"
- OSTEOPOROSIS INTERNATIONAL, Bd. 4, 1994, Seiten 21-31, XP000605170 WONG M.M. ET AL: "In vitro study of osteoblastic cells from patients with idiopathic osteoporosis and comparison with cells from non-osteoporotic controls"
- CALCIFIED TISSUE INTERNATIONAL , Bd. 54, 1994, Seiten 1-6, XP000605179 KASSEM M ET AL: "Human marrow stromal osteoblast-like cells do not show reduced responsiveness to in vitro stimulation with growth hormone in patients with postmenopauseal osteoporosis"
- THE JOURNAL OF CLINICAL ENDOCRINOLOGY AND METABOLISM, Bd. 69, Nr. 2, 1989, Seiten 272-279, XP000605184 PIERRE J. MARIE ET AL: "Osteocalcin and deoxyribonucleic acid synthesis in vitro and histomorphometric indices of bone formation in postmenopauseal osteoporosis"
- ANNALS OF MEDICINE, Bd. 25, 1993, Seiten 385-393, XP000605177 LEILA RISTELI ET AL: "biochemical markers of bone metabolism"

## Beschreibung

Die Erfindung betrifft ein Verfahren zur Osteoporosediagnose, mit dem eine zu 95% verläßliche Aussage zum Vorhandensein einer Osteoporose getroffen werden kann, und ein Verfahren zur Testung potentieller Osteoporose-Therapeutika, mit dem für jeden individuellen Fall ein wirksames Osteoporose-Therapeutikum ermittelt werden kann.

Osteoporose (Knochenschwund) ist eine schwere systemische Erkrankung des Skeletts, die sich durch mosaikartig auftretende verringerte Knochendichte (Masse) und mikroarchitektonische Veränderungen im Knochengewebe auszeichnet. Das Knochengewebe eines gesunden erwachsenen Menschen wird auch nach Beendigung seiner Entwicklung ständig weiter auf- und abgebaut. Die zwei entgegengesetzten Prozesse befinden sich normalerweise im Gleichgewicht. Überwiegt jedoch die Abbauphase (Knochenresorption), kommt es zunächst zum Knochenschwund - der Osteoporose-, einer verminderten Knochenfestigkeit. Die Erkrankung geht einher mit meist lebenslangen Schmerzen, gehäuften Frakturen, die begleitende Komplikationen bis hin zu tödlichen Verläufen nach sich ziehen können. Es wird geschätzt, daß weltweit über 200 Millionen, in der Bundesrepublik 7-8 Millionen, Menschen an dieser Krankheit leiden.

Die zur Zeit eingesetzten Therapeutika gegen Osteoporose, wie Östrogene, Progesterone, Calcitonin, Di/Bisphosphonate und Calcium können in ihrer Wirkung als Anti-Bone-Resorbers den Knochenschwund nur verlangsamen oder verringern. Hiermit gelingt es nicht, die bereits verlorene Knochensubstanz zu ersetzen.

Die Wirkung folgender Faktoren auf das Knochenwachstum wird zur Zeit untersucht:
Parathyroidhormon (PTH) und seine Derivate, Vitamin D3, anabole Steroide, Fluoride, Insulin-ähnliche Wachstumsfaktoren I und II, Prostaglandine und Wachstumshormon (GH). Die bisher veröffentlichten Ergebnisse der klinischen Studien sind bei weitem nicht zufriedenstellend, da bei den meisten Faktoren der Zuwachs an Knochendichte nur 1 bis 3% pro 1 - 3 Jahre beträgt (klinische Symptome der Osteoporose zeigen sich häufig erst nach dem Verlust von 50% der Knochenmasse) und die Frakturraten häufig nicht verringert werden.
Um also einem massiven Fortschreiten der Krankheit rechzeitig entgegenwirken zu können, spielt die Früherkennung der Osteoporose sowie die Osteoporosediagnose überhaupt eine wichtige Rolle.

Die zur Zeit eingesetzten diagnostischen physikalischen Methoden wie single photon absorptiometry (SPA), single- und dual X-ray absorptiometry (SXA; DXA) und quantitative computed tomographie (QCT) sind teuer, sie stehen nur in größeren klinischen Zentren zur Verfügung und sind äußerst schwierig zu interpretieren. Die Ultraschalluntersuchungen sowie X-ray photodensitometry sind kostengünstige Methoden, jedoch mit ähnlichen Nachteilen behaftet.

Die zur Zeit eingesetzten klassischen biochemischen Methoden zur Osteoporose-Diagnostik basieren auf der Bestimmung des Hydroxyprolins im Urin, der Calciumexkretion, der Alkalischen Phosphatase und des Osteocalcins im Serum. Die Bestimmung dieser Parameter im Serum ist unspezifisch, da die gemessenen Werte hoch variabel sind.

Die neuen Methoden zur Schätzung der Knochenresorption, wie die Messung des Deoxypyridinolins im Urin, bzw. die Methoden zur Bestimmung der Knochenbildung, wie die Messung der knochenspezifischen Alkalischen Phosphatase und der Prokollagen-Peptide im Serum, sollten mehr Informationen geben.

Pierre J. Marie (1991), J. Clinical Investigation 88: 1167-1172, beschreibt die Bestimmung der Zellproliferationsrate der Osteoblasten und der Osteocalcin- und Alkalische Phosphatase- Synthese in osteoporotischen Patienten und vergleicht diese Parameter mit denen aus Osteoblastenkulturen nicht osteoporotischer Probanden.

Kassem M et al. (1994), Calcified Tissue International 54: 1-6, beschreiben des Effekts der Wachstumshormorie auf die Osteoblastenzell-Proliferationsrate osteoporotischer Patienten und auf die Alkalische Phosphatase-, Procollagen Type I- und Osteocalcin-Synthese durch Osteoblasten aus osteoporotischen Patienten.

Alle erwähnten Methoden haben jedoch den Nachteil, daß sie erst dann Hinweise liefern, wenn schon ein Teil der Knochenmasse verlorengegangen ist.

Aufgabe der Erfindung war es deshalb, ein kostengünstiges und für den Fachmann einfach handhabbares Verfahren zur Osteoporosediagnose zu entwickeln, daß in jedem individuellen Fall eine zu mindestens 95% sichere Zuordnung zur Gruppe der an Osteoporose Erkrankten oder nicht an Osteoporose Erkrankten bzw. zum Therapieerfolg durch zweifelsfrei interpretierbare und reproduzierbare Parameter liefert.

Die Aufgabe der Erfindung wird durch ein Verfahren gemäß Anspruch 1 gelöst.

Es wurde gefunden, daß erfindungsgemäße Osteoblastenzellen aus osteoporotischen Patienten pathologische Veränderungen in ihrer Proliferation und ihrer Differenzierung im Vergleich zu den Osteoblastenzellen aus nicht osteoporotischen Patienten zeigen. Mit der Diskriminanzanalyse werden die Daten über die Proliferationsrate und der Expressionsintensität von osteoblastenspezifischen Differenzierungsmarkern mit einer Genauigkeit von über 95% den Osteoporose- und Nicht-Osteoporose-Patienten zugeordnet. Dabei unterscheiden sich an Osteoporose Erkrankte von nicht an Osteoporose Erkrankten in höchst signifikanter Weise.
Überraschenderweise sind diese Zellkulturen auch zur Testung potentieller Osteoporose-Therapeutika sehr gut geeignet, wodurch es möglich ist, für jeden Patienten ein individuell wirksames Therapeutikum zu ermitteln.

Die Knochenzellkulturen werden hergestellt, indem man aus transiliakalen Knochenbiopsien osteoporotischer Probanden die Osteoblasten-Vorläuferzellen durch sequenziellen enzymatischen Verdau gewinnt, diese kultiviert und als standardisierte Zellkulturen (gleiche Zellpopulation) etabliert. Die osteologischen Knochenbiopsien werden aus klinisch charakterisierten Patienten vorzugsweise aus dem Beckenkamm oder auch aus anderen Knochen entnommen (Knochenstanzen). Die ca. 1 - 2 cm langen und 1 - 5 mm dicken Knochenbiopsien werden mechanisch von eventuell vorhandenem Fett- und Bindegewebe getrennt, in mehrere Stücke aufgeteilt, mehrmals enzymatisch (vorzugsweise mit Collagenase) behandelt und die isolierten Osteoblasten-Vorläuferzellen als Monolayer kultiviert. Parallel dazu können dreidimensionale Zellkulturen auf denaturierten Kollagen Typ 1-Schwämmchen angelegt werden. Daraus entstehen standardisierte Knochenzellkulturen. Die primären Knochenzellen, die aus nicht-osteoporotischen Patienten gleichen Geschlechts und etwa gleichen Alters gewonnen werden, dienen als Kontrolle.

Das Zellkulturmedium besteht vorzugsweise aus α-MEM-Medium und HAM-F12-Medium (von Gibco) und enthält gegebenenfalls einen Serumzusatz. Das Verhältnis aus α-MEM-Medium und HAM-F12-Medium beträgt vorzugsweise 1:2, aber auch Verhältnisse zwischen 3:1 bis 1:3 führen zum gewünschten Ergebnis. Als Serumzusatz, der zwischen 1 - 12% betragen kann, dient fetales Kälberserum, humanes Serum, bovines Serumalbumin oder Ultroser. Bevorzugt werden 2 - 7% Ultroser, besonders bevorzugt 2 - 5% Ultroser, eingesetzt.

Erfindungsgemäß ist es durch die Wahl der beschriebenen Zellkulturbedingungen gelungen, die standardisierten Osteoblastenzellkulturen osteoporotischer Patienten über Monate bis zu 2 Jahren lebensfähig zu halten, wobei das erfindungsgemäße Diagnoseverfahren mit den Zellkulturen bis zur 2. Passage durchgeführt wird, da diese die Situation im menschlichen Körper am besten widerspiegeln.

Die aus primären Osteoblastenzellkulturen gewonnenen Zellextrakte wurden mittels hochauflösender 2D-Gelelektrophorese und Silver-Staining untersucht und mit den aus Osteoblastenzellkulturen nicht-osteoporotischer Patienten gewonnenen Zellextrakten verglichen.

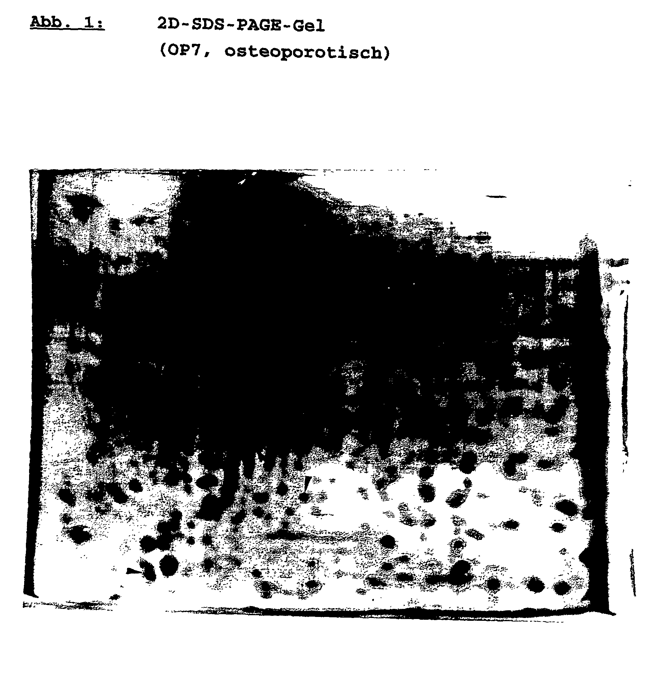

Es wurde gefunden, daß die Zellkulturen osteoporotischer Patienten im hochaufgelösten 2D-SDS-PAGE-Gel ein typisches, reproduzierbares Expressionsmuster von ca. 700 intrazellulären Proteinen gemäß der Spotverteilung in Abb. 1 zeigen.

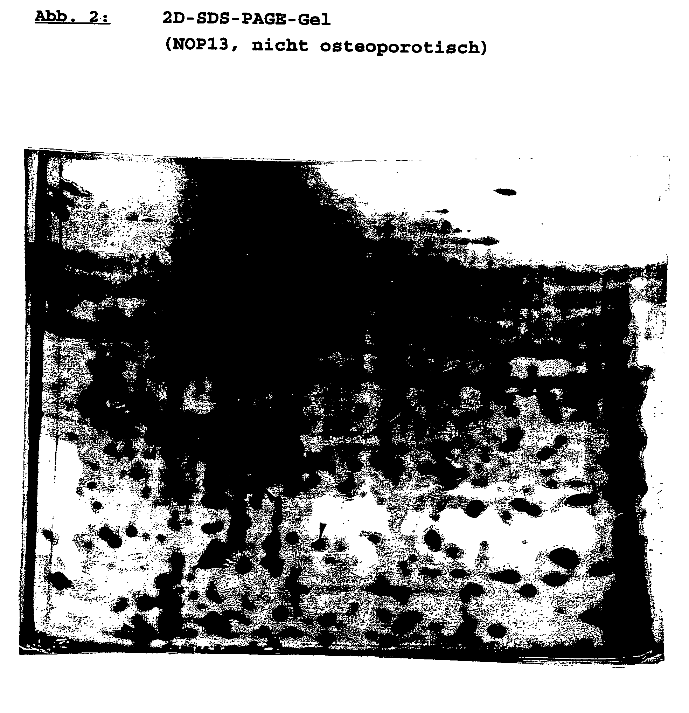

Dieses Proteinexpressionsmuster ist mit dem Proteinexpressionsmuster aus Osteoblastenzellkulturen nicht-osteoporotischer Patienten identisch (vgl. Abb. 2). Beim Vergleich der Intensitäten der einzelnen Spots in Abb.1 und in Abb. 2 zeigten sich jedoch reproduzierbare und quantitativ meßbare Unterschiede in mindestens sechs Spots (vgl. Kennzeichnung durch Pfeile in Abb. 1 und Abb. 2). Dabei ist eine erniedrigte Expression von fünf Proteinen und eine erhöhte Expression von einem Protein in den erfindungsgemäßen Osteoblastenzellkulturen gegenüber den Osteoblastenzellkulturen nicht-osteoporotischer Patienten festzustellen. Über die Intensitäten der Spots ist damit eine ergänzende Aussage über das Vorliegen einer Osteoporose möglich.

Die gewonnenen Proteinexpressionsmuster der Osteoblastenzellkulturen wurden auch mit denen von humanen Fibrosarkomen und humanen Osteosarkomen verglichen. Es zeigte sich, daß sich die Proteinexpressionsmuster der Fibrosarkome und Osteosarkome von dem Expressionsmuster der Osteoblasten unterscheiden. Dadurch wurde deutlich, daß es sich bei den erfindungsgemäßen Osteoblastenzellkulturen um Reinkulturen handelt, die nicht durch andere Zellarten verunreinigt sind.

Das erfindungsgemäße Verfahren zur Osteoporosediagnose basiert auf der Bestimmung geeigneter osteoblastenspezifischer Differenzierungsmarker, die Aussagen zur frühen Differenzierungsphase der Osteoblasten, zur Biosynthese und Reifung der extrazellulären Matrix und zur späten Differenzierungsphase der Osteoblasten erlauben. Es wurde gefunden, daß man zum Erhalt einer zu mindestens 95% zuverlässigen Aussage die Zellproliferationsrate und die Expression von mindestens sechs osteoblastenspezifischen Differenzierungsmarkern quantitativ bestimmen sollte, wobei man neben der Zellproliferationsrate noch mindestens einen Parameter der frühen Differenzierungsphase der Osteoblasten bestimmen sollte, mindestens einen Parameter der späten Differenzierungsphase und mindestens vier Parameter der Matrixsynthese. Vorzugsweise werden zur Untersuchung der frühen Differenzierungsphase neben der Zellproliferationsrate die Expression einer der drei Onkogene c-myc, c-fos oder c-jun, insbesondere c-fos, bestimmt, zur Untersuchung der Bildung und Reifung der extrazellulären Matrix die Expression der intrazellulären alkalischen Phosphatase, des Kollagen Typ I und Typ IV und von Chondroitinsulfat oder Hyaluronsäure und zur Untersuchung der späten Differenzierungsphase die intrazelluläre Synthese von Osteocalcin.

Die Expressionsintensitäten dieser Parameter werden mit denen aus primären Osteoblastenzellkulturen nicht-osteoporotischer Probanden gleichen Geschlechts und etwa gleichen Alters verglichen und statistisch ausgewertet, zum Beispiel mittels Diskriminanzanalyse. Selbstverständlich können zur weiteren Erhöhung der Aussage - Zuverlässigkeit zusätzliche Differenzierungsmarker wie zum Beispiel Wachstumsfaktoren (z.B. TGF β), Cytokine (z.B. IGF I, IGF II), in den Zellüberstand sekretierte alkalische Phosphatase sowie sekretiertes Kollagen Typ I und Typ IV bestimmt werden.

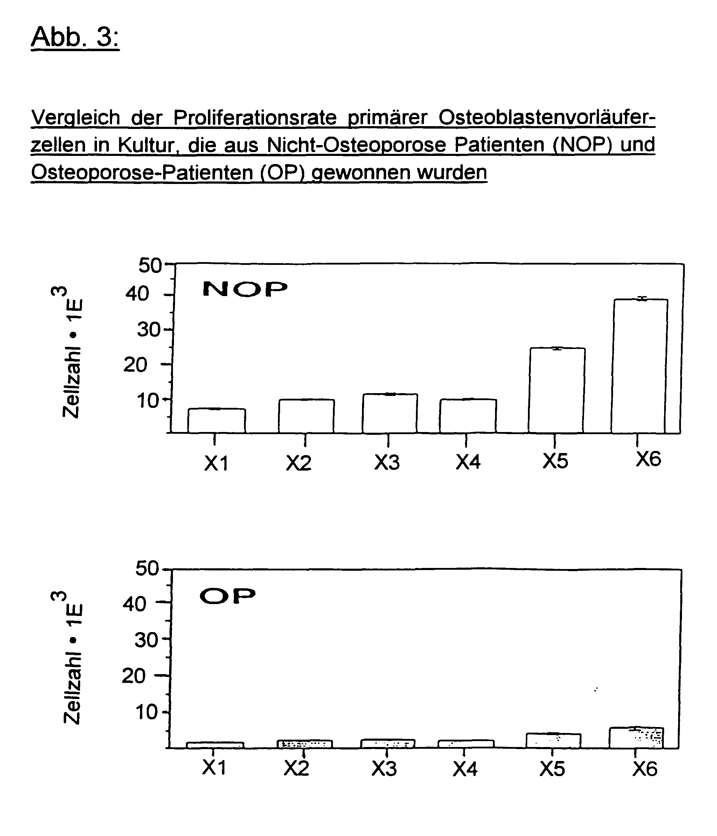

Zur Untersuchung der frühen Differenzierungsphase der Osteoblasten wird die Zellproliferationsrate in parallelen Kulturen nach 48 - 196 Stunden Inkubation, vorzugsweise nach 72 Stunden Inkubation, gemessen. Die Messung erfolgt mit üblichen Zellproliferationsassays, z.B. durch Einbau einer radioaktiv-markierten Substanz wie [³H]-Thymidin in die DNA. Auch der von der Fa. Boehringer Mannheim GmbH entwickelte Assay zum Einbau von Bromdesoxyuridin ist zur Bestimmung geeignet. Es zeigte sich, daß eine 4 - 6-fach verringerte Zellproliferationskapazität auf einen osteoporotischen Patienten hinweist (s. Abb. 3).

Daneben ist die Expression der drei Onkogene c-fos, c-myc und c-jun spezifisch für die frühe Differenzierung von Osteoblasten. Vorzugsweise wurde die Expression von c-fos bestimmt. Die Onkogenexpression wird nach der gleichen Inkubationszeit wie bei der Zellproliferationsrate mittels immunhistochemischer Färbung ihrer Proteinprodukte und Spektralphotometrie in den Monolayer-Zellkulturen quantitativ bestimmt.

Zur Untersuchung der frühen Differenzierungsphase der Osteoblasten kann zusätzlich die Quantifizierung der steady-state-level von jeweils spezifischer mRNA durch quantitative PCR und eventuell Northern blot-Analyse (falls die Zellzahl es erlaubt) durchgeführt werden.

Die Zellproliferationsrate und die Onkogen-Expressionsintensität in den osteoporotischen und nicht-osteoporotischen Zellen werden verglichen und eine Aussage zum Vorliegen von Osteoporose getroffen, beziehungsweise diese Parameter zusammen mit denen aus der späten Differenzierungsphase und der Matrixsynthese einer statistischen Auswertung unterzogen.

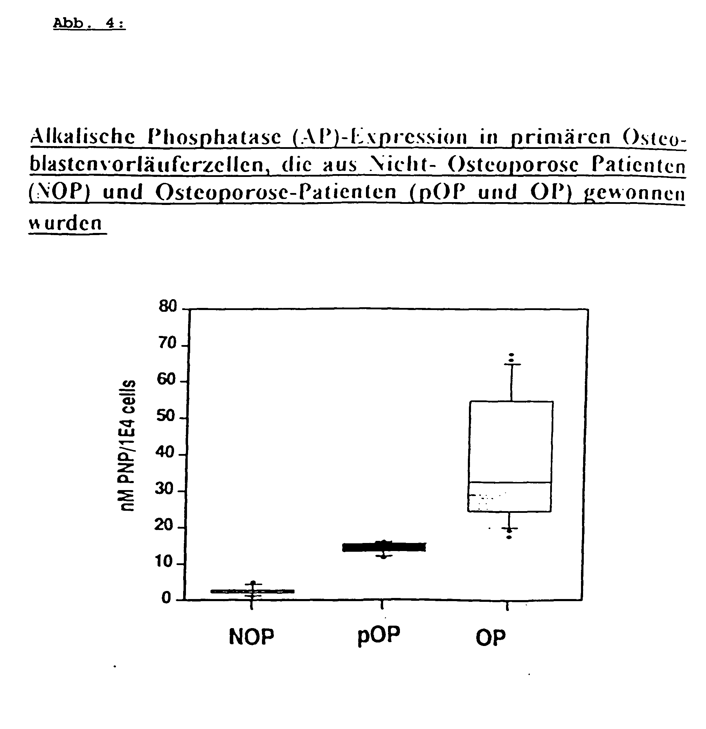

Bei der Untersuchung der Biosynthese und Reifung der extrazellulären Matrix, die durch osteoporotische und nicht-osteoporotische Zellen produziert wird, werden nach 3 bis 14 Tagen Inkubationszeit, vorzugsweise nach 3 und 7 Tagen, mindestens die intrazelluläre alkalische Phosphatase, Kollagen Typ I und Typ IV, Chondroitinsulfat oder Hyaluronsäure mittels immunhistochemischer Färbung ihrer Proteinprodukte und Spektralphotometrie quantitativ bestimmt. Es zeigte sich, daß Osteoblasten aus osteoporotischen Patienten 4 - 5mal mehr alkalische Phosphatase exprimieren als Osteoblasten gesunder Patienten (s. Abb. 4). Membrangebundenes Chondroitinsulfat wird von Osteoblasten aus osteoporotischen Patienten ebenfalls stärker exprimiert.

Die erfindungsgemäße Bestimmung der Expression der alkalischen Phosphatase in Osteoblastenzellkulturen osteoporotischer und nicht-osteoporotischer Patienten erlaubt es, nicht nur Aussagen zu einer bereits manifesten, sondern auch über eine beginnende Osteoporose zu treffen.
Eine beginnende Osteoporose zeichnet sich dadurch aus, daß vorzugsweise die endogene Expression der alkalischen Phosphatase leicht erhöht ist, bei einer manifesten Osteoporose dagegen ist die endogene Expression der alkalischen Phosphatase stark erhöht.

Die quantitative Bestimmung von Wachstumsfaktoren wie z.B. TGF β, insbesondere TGF β₂, und Cytokinen, wie z.B. IGF I und IGF II, mittels immunhistochemischer Färbung ihrer Proteinprodukte und Spektralphotometrie kann neben den genannten Parametern der weiteren Untersuchung der Bildung und Reifung der extrazellulären Matrix dienen.

Die Bestimmung der BMP's (bone morphogenetic proteins) in den osteoblastischen Zellen kann zusätzlich Informationen über die Erkrankung geben.

Die jeweils spezifische mRNA kann zur Untersuchung der Bildung und Reifung der extrazellulären Matrix ebenfalls zusätzlich durch quantitative PCR und Northern blot bestimmt werden.

Die in den Zellkulturüberstand sekretierte alkalische Phosphatase und Kollagen Typ I und IV können gegebenenfalls als zusätzliche Parameter quantifiert werden.

Zur Untersuchung der späten Differenzierungsphase der Osteoblasten (dem Mineralisationsprozeß) werden den parallelen Knochenzellkulturen mineralisierungsfördernde Reagenzien zugesetzt und die intrazelluläre Synthese von Osteocalcin quantitativ bestimmt. Diese Bestimmung erfolgt vorzugsweise mittels immunohistochemischer Färbung und Spektralphotometrie.

Der Nachweis Osteocalcin-spezifischer-mRNA kann zusätzlich durchgeführt werden und erfolgt mittels quantitativer PCR.

Als zusätzliche Maßnahme kann in der späten Differenzierungsphase auch neu synthetisierte Mineralmatrix und die Bildung der Mineralisierungsknoten durch Färbung mit Silbernitrat nach VAN KOSSA nachgewiesen werden.

Nachdem die als Minimum genannten 7 Parameter (Zellproliferation, ein Onkogen, alkalische Phosphotase, Kollagen Typ I und IV, Chondroitinsulfat oder Hyaluronsäure, Osteocalcin) bestimmt sind, erfolgt die Auswertung durch Gegenüberstellung mit denen aus nicht-osteoporotischen Zellen und gegebenenfalls statistisch, wobei sich hier die Diskriminanzanalyse als besonders geeignet erwiesen hat.
Es zeigte sich, daß die Proliferationsrate der Zellen, die aus Osteoporose-Patienten gewonnen wurden, in signifikanter Weise niedriger ist im Vergleich zu der Proliferationsrate der gesunden Patienten. Ist die c-fos-Onkogen- und TGF β₂-Expression der Zellen, die aus Osteoporose-Patienten gewonnen wurden, in signifikanter Weise niedriger und die Chondroitinsulfat- und Osteocalcin-Expression höher im Vergleich zur Expressionsrate der jeweiligen Differenzierungsmarker aus Zellen von nicht-osteoporotischen Patienten, so handelt es sich hierbei um eine osteoporotische Erkrankung. Die Syntheserate der alkalischen Phosphatase in den Osteoblasten, die aus Osteoporose-Patienten gewonnen wurden, liegt signifikant höher als die alkalische Phosphatase-Expression in den Osteoblasten von nicht-osteoporotischen Kranken.

Somit erlaubt das erfindungsgemäße Verfahren bereits durch die Bestimmung der genannten 7 Parameter, die mit für den Fachmann üblichen Methoden gemessen werden, eine zu 95% verläßliche Aussage zum Vorhandensein einer Osteoporose basierend auf den erfindungsgemäßen Zellkulturen.

Überraschenderweise zeigte sich, daß die Zellkulturen auch hervorragend zur Testung potentieller Osteoporose-Therapeutika geeignet sind und damit ein in vitro-Testsystem zur Verfügung steht, mit dem die direkte Wirkung von potentiellen Therapeutika auf individuelle menschliche Osteoblasten-Vorläuferzellen von gesunden Probanden und Patienten untersucht werden kann.

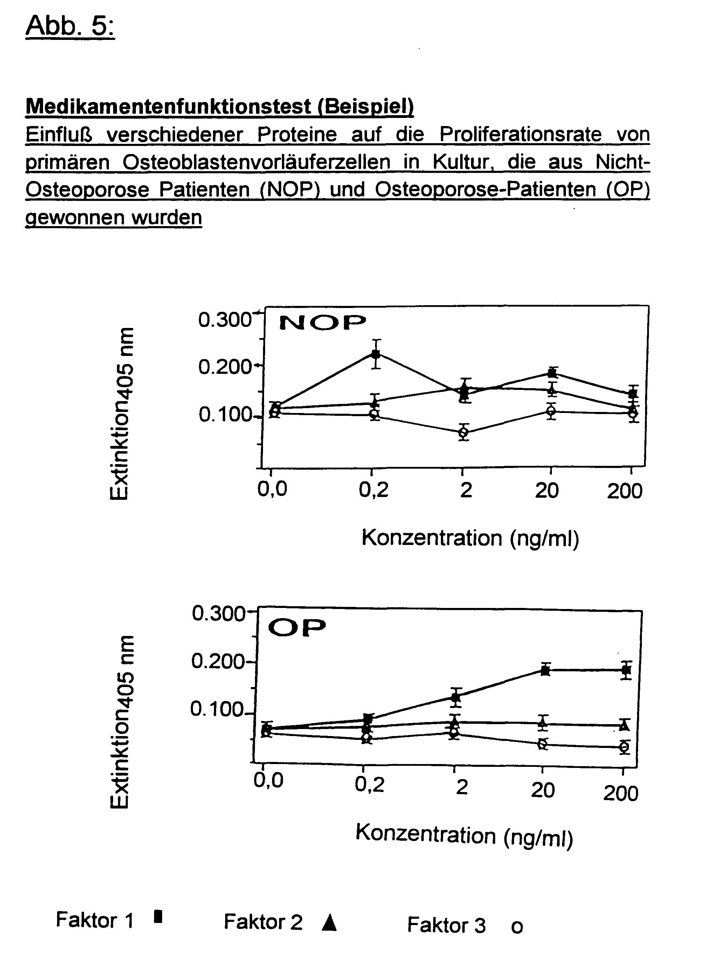

Gegenstand der vorliegenden Erfindung ist somit auch das Verfahren zur Testung potentieller Osteoporose-Therapeutika gemäß Anspruch 3.
Es zeigte sich, daß auch die mitogene Wirkung potentieller Osteoporose-Therapeutika durch Messung der Zellproliferationsrate mit den beschriebenen üblichen Methoden bestimmbar ist (vgl. Abb. 5).

Die Wirkung potentieller Osteoporose-Therapeutika auf die Differenzierung der "osteoporotischen" Osteoblasten-Vorläuferzellen kann ebenfalls durch die Messung der für die Osteoporose-Diagnose oben beschriebenen Mindestparameter bestimmt werden. Auch hier erhält man bereits durch Bestimmung der genannten Parameter mittels immunhistochemischer Färbung und Spektralphotometrie eine zuverlässige Aussage.

Zusätzlich kann der Einfluß potentieller Osteoporose-Therapeutika auf die Regulation der Matrixsynthese über den Nachweis spezifischer mRNA durch quantitative PCR bestimmt werden. Diese Bestimmung erfolgt derart, daß die gesamte RNA aus den Zellen isoliert wird, aus mRNA mittels oligo und/oder random primers und reverser Transkriptase eine cDNA Bank hergestellt wird und die bestimmte Menge jeweils spezifischer Einzelstrang - cDNA mittels spezifischer Primer-Paare amplifiziert wird. Zur Quantifizierung wird parallel das β-ACTIN Fragment als HOUSE-KEEPING Gen mittels PCR amplifiziert.

Dieser Test ergänzt in hervorragender Weise die z.Z. eingesetzten Tiermodelle zur Untersuchung von Osteoporose-Therapeutika, die nur eingeschränkt dem Krankheitsbild beim Menschen entsprechen können und keinesfalls eine individuelle Ermittlung besonders geeigneter Therapeutika erlauben. Werden die Knochensubstanzen von Patienten mit anderen skeletalen Erkrankungen entnommen, so ist es möglich, mit den daraus erfindungsgemäß etablierten Zellkulturen die Wirkung von potentiellen Therapeutika auch gegen diese skeletalen Erkrankungen zu testen.

Die standardisierten primären Osteoblastenzellkulturen osteoporotischer Patienten können durch das beschriebene Verfahren reproduzierbar gewonnen und bis zu zwei Jahren lebensfähig gehalten werden. Sie stellen Reinkulturen dar, die mittels hochauflösender 2D-SDS-Gelelektrophorese eindeutig identifizierbar und von den Osteoblastenzellkulturen nicht-osteoporotischer Patienten unterscheidbar sind.

Nachfolgend soll die Erfindung durch Abbildungen und Ausführungsbeispiele näher erläutert werden, ohne sie darauf einzuschränken.

### Abbildungen:

- Abb. 1:: Hochaufgelöstes 2D-SDS-PAGE-Gel standardisier- ter, primärer Osteoblastenzellkulturen osteopo- rotischer Patienten
Expressionsmuster der intrazellulären Proteine gemäß Spotverteilung
Pfeile: Intensitätsunterschiede von sechs Proteinen im Vergleich mit Abb. 2
- Abb. 2:: Hochaufgelöstes 2D-SDS-Page-Gel standardisier- ter, primärer Osteoblastenzellkulturen nicht- osteoporotischer Patienten
Expressionsmuster der intrazellulären Proteine gemäß Spotverteilung
Pfeile: Intensitätsunterschiede von sechs Proteinen im Vergleich mit Abb. 1
- Abb. 3:: Nachweis der verminderten Proliferationsrate von Osteoblastenvorläuferzellen osteoporo- tischer Patienten (OP-Zellen) im Vergleich zur Proliferationsrate von Osteoblastenvorläufer- zellen nicht-osteoporotischer Patienten (NOP- Zellen)
x1 - x3: Behandlung mit FCS (fetalem Kälberserum) 48 h, 120 h, 192 h
x4 - x6: Behandlung mit inaktiviertem humanen Kontrollserum, 48 h, 120 h, 192 h
(vgl. Beispiel 3)
- Abb. 4:: Nachweis der erhöhten Expression intrazellulä- rer alkalischer Phosphatase in Osteoblastenvor- läuferzellen von osteoporotischen Patienten (pOP- und OP-Zellen)
pOP: präklinische Osteoporose , n= 12
OP: Osteoporose, n=14
NOP: nicht-osteoporotisch, n=18
10 % FCS, 192 h
- Abb. 5:: Unterschiedliche Dosiswirkungskurven (Stimu- lierbarkeit) der Proliferation von NOP- und OP- Zellen durch Proteine (Wachstumsfaktoren 1, 2 und 3).
Es wird eine signifikante dosisabhängige mitogene Wirkung des Wachstumsfaktors 1 (TGF β₂) auf osteoblastische Zellen aus Osteoporose-Patienten deutlich.
- Abb. 6:: Diskriminanzanalyse (25 Proben) von osteoblastischen Zellen von an Osteoporose erkrankten (OP) und nicht erkrankten (NOP) Patienten

### Ausführungsbeispiele

### 1. Zellkultivierung

Das Zellkulturmodell basiert auf primären Zellkulturen, die aus Beckenkammbiopsien differenzialdiagnostisch charakterisierter Patienten mit Verdacht auf Osteoporose gewonnen wurden. Die Beckenkammbiopsien stammten von jeweils 26 osteoporotischen und 18 nicht-osteoporotischen Patientinnen im Alter von 50-70 Jahren.

Alle Knochenstanzen wurden wie folgt behandelt:
- Nach vorangegangenem Spülen der Stanze mit PBS und Befreiung von eventuell vorhandenem Fett- und Bindegewebe wurde das Knochenstück fünfmal mit verdünnter Collagenase-Lösung (0,5 mg/ml) von Worthington (CLS 2) jeweils 30 min. bei 37°C behandelt.
   Zur Verdünnung der Collagenase-Lösung wurde im Verhältnis 1:2 α-MEM-Medium und HAM-F12-Medium von Gibco ohne Zusatz von Serum verwendet.
- Jede dabei gewonnene Fraktion wurde in serumhaltigem Medium (Medium wie oben beschrieben) gewaschen und in 3% Ultroser enthaltendem Medium aufgenommen und kultiviert.

### 2. Zellkulturbedingungen

- Über den gesamten Zeitraum des Versuches wurden die Zellen in Serum enthaltendem Medium bestehend aus gleichen Teilen α-MEM-Medium und HAM-F12-Medium von Gibco kultiviert, wobei zweimal wöchentlich das Medium gewechselt wurde.
- Die Zellen der Fraktionen 1 bis 3 wurden vermehrt und fortlaufend passagiert.

### 3. Bestimmung der Zellproliferationsrate

- 3000 osteoblastische Zellen der Fraktionen 1 bis 2 bis zur zweiten Passage, die aus osteoporotischen Patienten gewonnen wurden (OP-Zellen), je Vertiefung einer 96 well Mikrotiterplatte wurden in Anwesenheit von 10% FCS und von 10% inaktiviertem humanen Kontrollserum in Zellkulturmedium (α-MEM- und HAM'S F12-Medium 1:12) für 48 h, 120 h und 192 h inkubiert. Parallel dazu wurden 3000 osteoblastische Zellen nicht-osteoporotischer Patienten unter gleichen Bedingungen kultiviert. Die Anzahl intakter Zellen wurde mittels Trypan-Blau-Färbung und Auszählen in einer Zählkammer bestimmt.

### 4. Bestimmung der Zellproliferationsrate unter Einfluß der drei Wachstumsfaktoren 1, 2 und 3 mittels Bromdesoxyuridin-Einbau (BrdU-Einbau)

Wachstumsfaktor 1 = TGF β₂
   2 = IGF I
   3 = IGF II

- Für den Proliferationstest wurden die Zellen aus der Fraktion 1 und/oder 2 jeder Knochenstanze bis zur 2. Passage verwendet.
- Zur Anwendung kamen 3x10³ Zellen der ersten und/oder zweiten Passage/well einer 96 well-Platte und 200µl Medium.
- Die Zellen wurden drei Tage unter bereits beschriebenen Bedingungen kultiviert und danach für einen Tag im Medium ohne Serum bis zur Subkonfluenz weiterkultiviert.
- Danach wurden drei Wachstumsfaktoren 1, 2 und 3 in vier verschiedenen Endkonzentrationen (100, 10, 1 und 0,1 ng/ml in Medium) zu den Zellen gegeben.
- Als Kontrollen dienten Zellen in 10%igem FKS haltigem Medium und in 5% Ultroser enthaltendem Medium.
- Von allen Konzentrationen und Kontrollen wurden Tripletts angesetzt.
- Als Blankkontrollen dienten sechs wells mit Zellen, denen während des Proliferationstestes kein Bromdesoxyuridin dazugegeben wurde.
- Nach 54stündiger Inkubation mit den Wachstumsfaktoren begann der Proliferationstest (5-Bromo-2'-desoxy-uridine Labeling and Detection Kit III von Boehringer Mannheim) mit der Zugabe von 20 µl 1:90 in 1%igem BSA-Medium verdünnter Labeling-Solution und anschließender Inkubation für 18 Stunden.
- Der Proliferationstest erfolgte nach Vorschrift des Herstellers.
- Die Auswertung des Proliferationstestes erfolgte durch Messung der optischen Dichte (OD) bei 405 nm im ELISA-Reader. Die Extinktion wurde nach 60 min Farbentwicklung alle 5 min. 12malig gemessen.

Die erhaltenen Werte sind aus Tabelle 1 ersichtlich.

In Abb. 5 wurden die gemessenen Extinktionen der drei Wachstumsfaktoren in Abhängigkeit von der Konzentration dargestellt.

**Tab. 1:**

| Wirkung von TFGβ₂, IGF II und IFG I auf humane Osteoblasten-Vorläuferzellen | | |
|---|---|---|
| Wachstumsfaktor | Extinktionsmittelwerte | Standardabweichung |
| TGFβ₂ 100 ng/ml | 0,190 | 0,002 |
| 10 ng/ml | 0,190 | 0,002 |
| 1 ng/ml | 0,170 | 0,008 |
| 0,1 ng/ml | 0,150 | 0,004 |
| | | |
| IGFI 100 ng/ml | 0,140 | 0,008 |
| 10 ng/ml | 0,140 | 0,002 |
| 1 ng/ml | 0,148 | 0,006 |
| 0,1 ng/ml | 0,151 | 0,005 |
| | | |
| IGF II 100 ng/ml | 0,140 | 0,000 |
| 10 ng/ml | 0,139 | 0,001 |
| 1 ng/ml | 0,150 | 0,001 |
| 0,1 ng/ml | 0,148 | 0,004 |
| 10% FKS | 0,194 | 0,014 |
| 5% Ultroser | 0,157 | 0,003 |
| 3% Ultroser | 0,144 | 0,008 |
| 1 % BSA | 0,145 | 0,001 |
| Blank | 0,131 | 0,001 |

### 5. Durchführung der hochauflösenden 2D-SDS-Gelelektrophorese (IEF-SDS-PAGE)

Die 2D-SDS-Gelektrophorese der NOP- und OP-Zellextrakte wurde wie in "Electrophoresis 1994, 15, 685-707, S. 686" beschrieben, durchgeführt.
- Erste Dimension:: isoelektrische Fokussierung (IEF) (linke Seite des Gels: saure Proteine mit IP z.B. 4; rechte Seite des Gels: Proteine mit einem IP von z.B. 8)
- Zweite Dimension:: SDS-PAGE unter reduzierenden Bedingungen (Proteine mit kleinem Molekulargewicht erscheinen im unteren Bereich, mit großem Molekulargewicht im oberen)

Die Auswertung der Intensitäten der Spots erfolgte mittels Scanning Densitometry und statistisch.

### 6. Untersuchuncismethoden und Materialien Bestimmung der intrazellulären und sekretierten alkalischen Phosphatase (AP)

### Zellkulturbedingungen:

3000 osteoblastische Zellen der Fraktionen 1 und 2 bis zur zweiten Passage, die aus osteoporotischen Patienten gewonnen wurden (OP-Zellen), je Vertiefung einer 96-well Mikrotiterplatte, wurden in Anwesenheit von 10% FCS und von 10% inaktivierten humanen Kontrollserum in Zellkulturmedium (α - MEM - und HAM S F12- Medium) für 24h vorinkubiert. Parallel dazu wurden 3000 osteoblastische Zellen Nicht-osteoporotischer Patienten unter gleichen Bedingungen kultiviert.

### AP-Assay:

Die Zellen wurden drei Tage unter den beschriebenen Bedingungen kultiviert und anschließend der AP-Assay durchgeführt. Die Zellen werden mit Phosphatpuffer von Dulbecco 2x gewaschen und anschließend mit je 100 µl einer Lösung bestehend aus 0.1 M Glycin, pH 10.3, 1 mM ZnCl₂, 1mM MgCl₂, 0.1% Triton X-100 lysiert. In alle Vertiefungen einer Mikrotiterplatte wurden je 50 µl einer 2.5 mM Lösung an Di-Natrium-4-nitrophenylphosphat Hexahydrat (AP-Reagenz) hinzugegeben und die dabei auftretende Farbentwicklung innerhalb einer Stunde in Abständen von 15 min bei 405 nm in einem TITERTEK ELISA Reader gemessen. Die enzymatische Aktivität korreliert mit der Farbintensität und wird als nM von para-Nitrophenylphosphat als Substrat pro bestimmter Zellzahl dargestellt. Parallel wurde eine Verdünnungsreihe einer 1mM 4-Nitrophenollösung als Standard pipettiert. Als Kontrollen dienten Zellen in 10%-igem FCS-haltigem Medium.

### PCR:

### Amplifikation von mRNA, die osteoblastensipezifische Differenzierungsmarker kodieren, mittels Reverse-Transkriptase-Polymerase-Ketten-Reaktion (RT-PCR)

Die Amplifikation bestimmter mRNA-Sequenzen mittels RT-PCR erfolgt durch
a) Gesamt-RNA-Isolierung,
b) Synthese von Einzelstrang-cDNA durch Umschreibung von mRNA in cDNA mit Superscript II Reverse Transkriptase unter Anwendung von oligo-dTbeziehungsweise random primer,
c) Amplifikation von cDNA-Fragmenten, die spezifisch die Osteoblastendifferenzierungsmarker kodieren mittels PCR,
d) Nachweis spezifisch amplifizierter PCR-Produkte in Agarosegelen.

### Gesamt-RNA-Isolierung

Die Gesamt-RNA aus 1-2 Millionen Osteoblasten und osteoblast-like-cells wurde isoliert (Trizol-Kit, Gibco Life Technologies).

### Synthese von Einzelstrang-cDNA durch Umschreibung von m-RNA in c-DNA mit Superscript II Reverse Transkriptase unter Anwendung von oligo-dT- bzw. random primer

Bis zu einem halben Mikrogramm der Gesamt-RNA oder 5-50 ng der mRNA pro Probe wurden mit Superscript II Reverse Transkriptase unter Anwendung von 500 ng oligo-dT- bzw. 500 pg random primer (Superscript II Reverse Transkriptase Kit, Gibco, Life Technologies) bei 42°c für 50 min umgeschrieben.
Die daraus resultierende cDNA werden als Matrizen(template) für Amplifikation in PCR eingesetzt.

### Amplifikation von cDNA-Fragmenten, die spezifisch die Osteoblastendifferenzieruaasmarker kodieren mittels PCR

Ein Zehntel der Einzelstrang-cDNA Probe (s.o.) wurde in 2 mM Tris-HCl; pH 8,4; 5 mM KCl; 1,5 mM MgCl₂; 10 nM Amplifikationsprimer sense, 10 nM Amplifikationsprimer antisense unter folgenden PCR-Bedingungen (vorzugsweise) amplifiziert:
- Denaturation:: 10 min
- Acht PCR-Zyklen wurden wie folgt durchgeführt:: Denaturation 94°C, 45 Sek.,
- Annealing:: 58°C, 1 min, 45 Sek.; Extension bei 72°C, 3 min;
- 25 PCR-Zyklen folgten darauf:: Denaturation 94°C, 45 Sek.
- Annealing:: 55°C, 45 Sek.; Extension bei 72°C, 3min
- Extension:: 72°C, 10 min

### Gelektroiphorese von PCR-Produkten

Ein Zehntel bis ein Zwanzigstel des PCR-Produktes wurden in 0,7%- bis 1,2%-igem Agarosegel für eine halbe Stunde bei 60V konstanter Spannung aufgetrennt, und das Molekulargewicht amplifizierter PCR-Produkte mit einem DNA-Marker verglichen.

### Sense- und Antisense Primer-Sequenzen

Die Sequenzen der Primerpaare, die spezifisch für Osteoblastendifferenzierungsmarker-kodierende cDNA sind, wurden von der co.don GmbH mit Hilfe von MacMollyTetra Software (Prof. B. Wittig) ausgesucht.

### Northern Blotting

### Nachweis von Osteoblasten-Differenzierungsmarkerspezifischen mRNA:

Die Detektion bestimmter mRNA-Sequenzen durch Hybridisierung erfolgt durch die Auftrennung der Gesamt-RNA in einer Agarosegelmatrix, deren Übertragung und anschließende Fixierung auf ein Filter und Hybridisierung mit einer spezifischen DNA-Sonde.

### Gesamt-RNA-Isolierung

Die Gesamt-RNA aus 10-20 Millionen Osteoblasten und osteoblast-like-cells wurde isoliert (Trizol-Kit von Gibco).

### RNA-Gelektrophorese

Je fünf bis zehn Mikrogramm der Gesamt-RNA wurden in 1,2%-tigem Formaldehyd-Agarose Gel für eine Stunde bei 100 V konstanter Spannung aufgetrennt.

### Transfer

Nach der Elektrophorese wurden die Gele für eine Stunde auf eine amphotere Nytran-Membran (Schleicher & Schuell) über Nacht geblottet und anschließend die Nukleinsäuren durch die UV-Strahlung vernetzt.

### Markieren der DNA-Sonde

Je ein cDNA-Fragment, das 40 Basen lang ist und spezifisch für die zu untersuchenden OsteoblastenDifferenzierungsmarker, wurde mittels Polymerase-Ketten-Reaktion (PCR) ampfliziert und anschließend mit Biotin -Reagenz Bio-ULS (Dianova GmbH) für eine Stunde bei 87°C inkubiert.

### Hybridisierung

Die Nitrozellulose-Membran wurde nach UV-Vernetzung mit 75 mM Natriumzytratpuffer, 750 mM NaCl, 5% Polyvinylpyrolidone, 0,1% BSA, 5 mM EDTA, 0,5% SDS, 0,1 mg/ml Heringspermien-DNA bei 42°C 15 min prähybridisiert und danach bei 42°C mit einer Lösung von 50% Formamid, 1% Rinderserumalbimin, 1mM EDTA, 0,5 mM Natrium-Phosphat und 5% Natrium-Dodecylsulfat für 16 Stunden mit spezifischer Biotin-markierter DNA-Sonde (ca. 100 ng) inkubiert.

### Detektion von mRNA

Die Membran wurde nach Hybridisierung gewaschen, mit 1,5% Trockenmilchlösung geblockt und mit einer Streptavidin/alkalische Phosphatase (Schleicher & Schuell) Lösung für 15 min bis 3 Stunden inkubiert. Die Membran wurde anschließend mit 0,5% Tween-20, PBS-Lösung für 5 min gewaschen und die Farbbildung erfolgte durch eine Inkubation der Membran in einer 0,1 M Tris-HCl; 0,1 M NaCl; 5 mg MgCl, NBT (32 mg/ml) in 70% Dimethylformamide, BCIP (16 mg/ml) für eine Stunde bis 16 Stunden.

### Primer/DNA-Sonden Sequenzen

Die Sequenzen der Primerpaare und/oder DNA-Hybridisierungssonden, die spezifisch für Osteoblastendifferenzierungsmarker-kodierende cDNA sind, wurden mit Hilfe von MacMollyTetra Software (Prof. B. Witting) ausgesucht.

### Durchführung der immunhistochemischen Färbung

- Prinzip:: Detektion der zellulären Antigene in einem konfluenten Zellrasen mittels spezifischer Antikörper über einen zweiten Antikörper (Enzym- oder Biotin-Konjugat) und nachfolgendem Nachweis durch Substratspaltung

- Zellen werden in einer Konzentration 1x10³ bis 5x10⁴ Zellen/well ausgesät und solange im Brutschrank bei 37°C inkubiert, bis sich ein konfluenter Zellrasen gebildet hat
- Medium absaugen und 3x mit PBS waschen
- Fixieren der Zellen: 100 µl eiskaltes Methanol/well zugeben und 10 bis 20 Min. bei 4°C inkubieren,
   3x mit PBS waschen
- Absättigen der freien Bindungsstellen im well:
   100 µl 2% Magermilch in PBS/well zugeben und 1 Std. bei Raumtemperatur (RT) oder über Nacht bei 4°C schütteln, Platte ausklopfen oder absaugen
- 50 µl des jeweils spezifischen Antikörpers zugeben und 1 Std. bei RT oder über Nacht bei 4°C schütteln, 3x mit PBS waschen
- 50 µl des 2. Antikörpers (Peroxidase-Konjugat; es werden die spezifischen Antikörper goat-anti-rabbit oder goat-anti-mouse von Sigma verwendet) zugeben und 1 Std. bei RT oder über Nacht bei 4°C schütteln, 5x mit PBS waschen
- Substrat-Zugabe: 100 µl (löslich, ABTS) SubstratLösung
   (ABTS = 2,2'-Azino-bis-(3-Ethylbenzthiazolin-6-sulfonsäure) Tablette lösen in 100 ml 0.05 M Phosphat-Citrat-Puffer, pH 5,0;
   (25,7 ml Di-natriumhydrogenphosphat + 24,3 ml Citronensäure) und 25 µl 30% Wasserstoffperoxid ad 100 ml mit destiliertem H₂O;
   60 Min. im Dunkeln inkubieren
- in well A12 100 µl Substrat geben (Background); Optische Dichte (OD) messen bei 405 nm im ELISA-Reader

Als spezifische Antikörper wurden eingesetzt:
- für Hyaluronsäure -: mouse-Anti-Hyaluronat-MAK (Camon/Serotec # MCA 277)
- für Chondroitinsulfat -: mouse-Anti-Chondroitinsulfat- MAK (Sigma # C 8035)
- für Kollagen Typ I -: mouse-Anti-Human-Collagen Typ I MAK (Chemicon # MAK 1340)
- für Kollagen Typ IV -: mouse-Anti-Collagen Typ IV- MAK (Sigma # C 1926)
- für TGF β -: mouse-Anti-TGF β-1,2,3-MAK (Genzyme # B 3026)
- für c-fos -: rabbit-Anti-c-fos-AK, polyclonal (Dianova # PC 05)
- für c-jun -: rabbit-Anti-c-jun-AK, polyclonal (Dianova # PC 07)
- für c-myc -: mouse-Anti-c-myc-MAK (Dianova # OP 10)
- für Osteocalcin -: rabbit-Anti-Human- Osteocalcin-AK (Paesel+Lorei # 14-143-0071)

### 7. Statistische Auswertung mittels Diskriminanzanalyse

Mit Hilfe der Diskriminanzanalyse wird eine Osteoblastenkultur aufgrund der zellulären Proliferationsrate und der Expressionsintensitäten zellulärer Differenzierungsmarker der Gruppe der an Osteoporose erkrankten Patienten oder der Gruppe der gesunden Individuen zugeordnet.
Ziel ist es, die Koeffizienten zu ermitteln, die die Werte der Diskriminanzfunktion beider Gruppen möglichst gut trennen.
Die Diskriminanzanalyse der osteoblastischen Zellen von an Osteoporose erkrankten und nicht erkrankten Patienten, bei der die 7 erfindungsgemäßen getesteten Variablen einfließen, ergibt folgende Daten:

| | |
|---|---|
| Richtige Gruppenzuordnung | 95,1% |
| Koeffizient der Diskriminanzanalyse | 0,8217 |
| Signifikanz p der Diskriminanz | 0,0000 |
| Eigenvalue | 2,0792 |

Es ist anhand der Daten zu erkennen, daß eine sehr gute Trennung zwischen den NOP und OP erfolgt.

**Tabelle 2:**

| Klassifikationstabelle der Diskriminanzanalyse | | | |
|---|---|---|---|
| **Gruppe** | **Anzahl der Proben** | **Zuordnung zu OP** | **Zuordnung zu NOP** |
| osteoporotische Zellen | 25 | 24 (96,0%) | 1 (4,0%) |
| nichtosteoporotische Zellen | 16 | 1 (6,2%) | 15 (93,8%) |

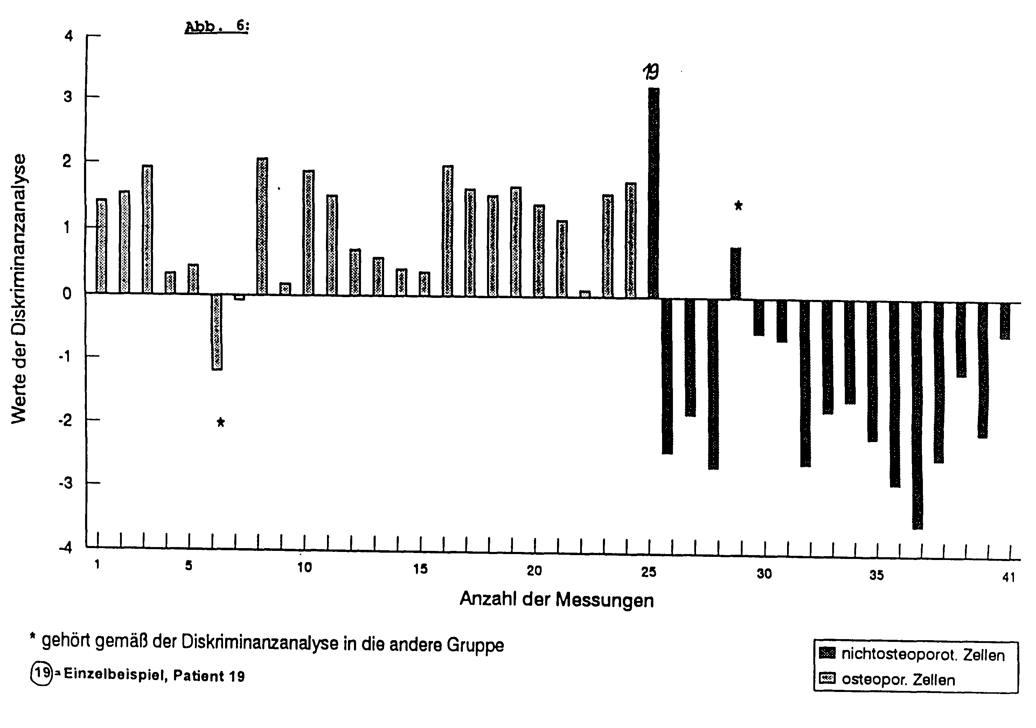

Der Eigenvalue, das Verhältnis der Quadratsumme zwischen den Gruppen zu der Quadratsumme innerhalb der Gruppen, ist sehr hoch. Hohe Eigenwerte zeigen gute Diskriminanzfunktionen an. Die Ergebnisse der Diskriminanzanalyse werden in der Abb. 6 gezeigt.

## Patentansprüche

1. Verfahren zur Osteoporosediagnose,
**dadurch gekennzeichnet,**
**daß** man zum Erhalt einer zu mindestens 95% verläßlichen Aussage in einem ersten Schritt standardisierte, primäre Osteoblastenzellkulturen in vitro herstellt, indem man durch sequenziellen enzymatischen Verdau transiliakaler Knochenbiopsien osteoporotischer Patienten, isolierte Osteoblasten-Vorläuferzellen gewinnt, diese kultiviert, anschließend
die Zellproliferationsrate und die Expression von mindestens sechs osteoblastenspezifischen Differenzierungsmarkern quantitativ mit an sich üblichen Methoden bestimmt, wobei man neben der Zellproliferationsrate noch mindestens einen Parameter der frühen Differenzierungsphase der Osteoblasten, ausgewählt aus der Expression einer der drei Onkogene c-myc, c-fos oder c-jun, bestimmt, als Parameter der späten Differenzierungsphase die intrazelluläre Synthese von Osteocalcin bestimmt und als Parameter der Matrixsynthese mindestens die Expression der intrazellulären alkalischen Phosphatase, des Kollagen Typ I, des Kollagen Typ IV und von Chondroitin-Sulfat oder Hyaluronsäure bestimmt und diese Parameter mit denen aus primären Osteoblastenzellkulturen nicht-osteoporotischer Probanden gleichen Geschlechts und etwa gleichen Alters vergleicht und gegebenenfalls statistisch auswertet.

2. Verfahren gemäß Anspruch 1,
**dadurch gekennzeichnet,**
**daß** man zur Untersuchung der frühen Differenzierungsphase der Osteoblasten neben der Zellproliferationsrate die Expression von c-fos bestimmt.

3. Verfahren zur Testung potentieller Osteoporose-Therapeutika,
**dadurch gekennzeichnet,**
**daß** man die mitogene Wirkung der potentiellen Wirkstoffe und/oder ihre Wirkung auf die Differenzierung der Osteoblasten-Vorläuferzellen in vitro in standardisierten, primären Osteoblastenzellkulturen osteoporotischer Patienten durch quantitative Bestimmung der Zellproliferationsrate und der Expression von mindestens sechs osteoblastenspezifischen Differenzierungsmarkern quantitativ mit an sich üblichen Methoden bestimmt, wobei man in einem ersten Schritt standardisierte, primäre Osteoblastenzellkulturen herstellt, indem man durch sequenziellen enzymatischen Verdau transiliakaler Knochenbiopsien osteoporotischer Patienten, isolierte Osteoblasten-Vorläuferzellen gewinnt, diese kultiviert und neben der Zellproliferationsrate noch mindestens einen Parameter der frühen Differenzierungsphase der Osteoblasten, ausgewählt aus der Expression einer der drei Onkogene c-myc, c-fos oder c-jun, bestimmt, als Parameter der späten Differenzierungsphase die intrazelluläre Synthese von Osteocalcin bestimmt und als Parameter der Matrixsynthese mindestens die Expression der intrazellulären alkalischen Phosphatase, des Kollagen Typ I, des Kollagen Typ IV und von Chondroitin-Sulfat oder Hyaluronsäure bestimmt und diese Parameter mit denen aus primären Osteoblastenzellkulturen nicht-osteoporotischer Probanden gleichen Geschlechts und etwa gleichen Alters vergleicht und gegebenenfalls statistisch auswertet.

4. Verfahren gemäß Anspruch 3,
**dadurch gekennzeichnet,**
**daß** man zur Untersuchung der frühen Differenzierungsphase der Osteoblasten neben der Zellproliferationsrate die Expression von c-fos bestimmt.

5. Verfahren nach Anspruch 1 oder 3,
**dadurch gekennzeichnet,**
**daß** die patientenspezifischen Osteoblastenzellkulturen in einem Zellkulturmedium, das aus α-MEM-Medium und HAM-F12-Medium besteht und gegebenenfalls einen Serumzusatz enthält, kultiviert werden.

6. Verfahren nach Anspruch 5,
**dadurch gekennzeichnet,**
**daß** die patientenspezifischen Osteoblastenzellkulturen in einem Zellkulturmedium, das 1 - 12% eines Serumzusatzes enthält und dessen Verhältnis von α-MEM-Medium zu HAM-F12-Medium zwischen 3:1 bis 1:3 beträgt, kultiviert werden.

## Claims

1. A method for diagnosing osteoporosis, **characterized in that** in order to obtain an at least 95% reliable statement, standardized, primary osteoblast cell cultures are prepared in vitro in a first step by sequential enzymatic digestion of transiliacal bone biopsies from osteoporotic patients and obtaining and cultivating isolated osteoblast precursor cells, afterwards the cell proliferation rate and expression of at least six osteoblast-specific differentiation markers are determined quantitatively using per se common methods, and in addition to the cell proliferation rate, at least one parameter of the early differentiation phase of the osteoblasts, selected from the expression of one of the three oncogenes c-myc, c-fos or c-jun is determined, the intracellular synthesis of osteocalcin is determined as parameter of the late differentiation phase, and at least the expression of intracellular alkaline phosphatase, collagen type I, collagen type IV and chondroitin sulfate or hyaluronic acid is determined as parameter of the matrix synthesis, and these parameters are compared to those of primary osteoblast cell cultures from non-osteoporotic test persons of same sex and about same age and optionally subjected to statistical evaluation.

2. The method according to claim 1, **characterized in that** in examining the early differentiation phase of the osteoblasts, the expression of c-fos is determined in addition to the cell proliferation rate.

3. A method for testing potential therapeutic agents for osteoporosis, **characterized in that** the mitogenic effect of the potential active substances and/or their effect on the differentiation of osteoblast precursor cells in vitro in standardized, primary osteoblast cell cultures from osteoporotic patients is determined by means of a quantitative determination of the cell proliferation rate and expression of at least six osteoblast-specific differentiation markers using per se common methods, wherein in a first step standardized, primary osteoblast cell cultures are prepared by sequential enzymatic digestion of transiliacal bone biopsies from osteoporotic patients and obtaining and cultivating isolated osteoblast precursor cells, and in addition to the cell proliferation rate, at least one parameter of the early differentiation phase of the osteoblasts, selected from the expression of one of the three oncogenes c-myc, c-fos or c-jun is determined, the intracellular synthesis of osteocalcin is determined as parameter of the late differentiation phase, and at least the expression of intracellular alkaline phosphatase, collagen type I, collagen type IV and chondroitin sulfate or hyaluronic acid is determined as parameter of the matrix synthesis, and these parameters are compared to those of primary osteoblast cell cultures from non-osteoporotic test persons of same sex and about same age and optionally subjected to statistical evaluation.

4. The method according to claim 3, **characterized in that** in examining the early differentiation phase of the osteoblasts, the expression of c-fos is determined in addition to the cell proliferation rate.

5. The method according to claim 1 or 3, **characterized in that** the patient-specific osteoblast cell cultures are cultivated in a cell culture medium which consists of α-MEM medium and HAM-F12 medium and optionally contains added serum.

6. The method according to claim 5, **characterized in that** the patient-specific osteoblast cell cultures are cultivated in a cell culture medium which contains from 1 to 12% of added serum and wherein the ratio of α -MEM medium to HAM-F12 medium is between 3:1 and 1:3.

## Revendications

1. Méthode de diagnostic de l'ostéoporose,
**caractérisée en ce**
**qu'**on prépare *in vitro* des cultures de cellules d'ostéoblastes primaires, normalisées dans une première étape pour obtenir une conclusion digne de foi à au moins 95 %, en obtenant des cellules précurseurs d'ostéoblastes isolées par digestion enzymatique séquentielle de biopsies de l'os transiliaques prélevées sur des patients atteints d'ostéoporose, en cultivant celles-ci, puis en déterminant quantitativement la vitesse de prolifération cellulaire et l'expression d'au moins six marqueurs de différenciation spécifiques aux ostéoblastes par des méthodes connues pour cela, moyennant quoi on détermine en plus de la vitesse de prolifération cellulaire encore au moins un paramètre de la phase précoce de différenciation des ostéoblastes, sélectionné parmi l'expression d'une des trois oncogènes c-myc, c-fos ou c-jun, on détermine en tant que paramètre de la phase tardive de différenciation la synthèse intracellulaire de l'ostéocalcine et on détermine en tant que paramètre de la synthèse de matrice au moins l'expression de la phosphatase alcaline intracellulaire, du collagène de type I, du collagène de type IV et du chondroïtine-sulfate ou de l'acide hyaluronique et qu'on compare ces paramètres avec ceux obtenus avec des cultures cellulaires d'ostéoblastes primaires provenant de sujets non atteints d'ostéoporose du même sexe et à peu près du même âge et on effectue éventuellement une évaluation statistique.

2. Méthode selon la revendication 1,
**caractérisée en ce**
**qu'**on détermine, pour étudier la phase précoce de différenciation des ostéoblastes, en plus de la vitesse de prolifération cellulaire, l'expression du c-fos.

3. Méthode destinée à tester les agents thérapeutiques potentiels contre l'ostéoporose,
**caractérisée en ce**
**qu'**on détermine l'effet mitogène des agents actifs potentiels et/ou leur effet sur la différenciation des cellules précurseurs d'ostéoblastes *in vitro* dans des cultures cellulaires d'ostéoblastes primaires, normalisées, prélevées sur des patients atteints d'ostéoporose, par une détermination quantitative du taux de prolifération cellulaire et de l'expression d'au moins six marqueurs de différenciation spécifiques aux ostéoblastes quantitativement par des méthodes connues pour cela, moyennant quoi on prépare des cultures de cellules d'ostéoblastes primaires, normalisées, dans une première étape, en obtenant des cellules précurseurs d'ostéoblastes isolées par digestion enzymatique séquentielle de biopsies d'os transiliaques prélevées sur des patients atteints d'ostéoporose, on cultive celles-ci, puis on détermine en plus de la vitesse de prolifération cellulaire encore au moins un paramètre de la phase précoce de différenciation des ostéoblastes, sélectionné parmi l'expression d'une des trois oncogènes c-myc, c-fos ou c-jun, on détermine en tant que paramètre de la phase tardive de différenciation la synthèse intracellulaire de l'ostéocalcine et on détermine en tant que paramètre de la synthèse de matrice au moins l'expression de la phosphatase alcaline intracellulaire, du collagène de type I, du collagène de type IV et du chondroïtine-sulfate ou de l'acide hyaluronique et on compare ces paramètres avec ceux obtenus avec des cultures cellulaires d'ostéoblastes primaires provenant de sujets non atteints d'ostéoporose du même sexe et à peu près du même âge et on effectue éventuellement une évaluation statistique.

4. Méthode selon la revendication 3,
**caractérisée en ce**
**qu'**on détermine, pour étudier la phase précoce de différenciation des ostéoblastes, en plus de la vitesse de prolifération cellulaire, l'expression du c-fos.

5. Méthode selon la revendication 1 ou 3,
**caractérisée en ce**
**qu'**on réalise les cultures cellulaires d'ostéoblastes spécifiques au patient dans un milieu de culture cellulaire qui est constitué d'un milieu α-MEM et d'un milieu HAM F12 et qui contient éventuellement un additif de sérum.

6. Méthode selon la revendication 5,
**caractérisée en ce**
**qu'**on réalise les cultures cellulaires d'ostéoblastes spécifiques au patient dans un milieu de culture cellulaire qui contient de 1 à 12 % d'un additif de sérum et dont le rapport du milieu α-MEM au milieu HAM F12 est compris entre 3 : 1 et 1 : 3.
